# EUROPEAN PATENT SPECIFICATION

(11) **EP 2 893 907 B1**
(45) Date of publication and mention of the grant of the patent: **29.11.2017**
(21) Application number: 14275261.7
(22) Date of filing: 17.12.2014
(51) Int. Cl.: A61F 2/966, A61M 25/04, A61M 25/06, A61F 2/07, A61M 39/06

(54) **A delivery device for an insertable medical device**
Einführvorrichtung für eine einführbare medizinische Vorrichtung
Dispositif de distribution pour un dispositif médical insérable

(30) Priority: 08.01.2014 AU 2014200124
(43) Date of publication of application: 15.07.2015
(73) Proprietor: Cook Medical Technologies LLC, Bloomington, IN 47404 (US)
(72) Inventor: Barrand, Zoe, Greenslopes, Queensland 4120 (AU); Ducke, Werner, Eight Mile Plains, Queensland 4113 (AU); Moran, Adrian, Co. Westmeath (IE); Yang, Mena, Browns Plains, Queensland 4118 (AU)
(74) Representative: Williams Powell

(56) References cited:
- US-A- 5 542 936
- US-A- 6 029 671
- US-A1- 2010 100 055
- US-A1- 2010 249 895
- US-B2- 6 743 196
- US-B2- 8 419 783

## Description

### TECHNICAL FIELD

This invention relates to a delivery device for an insertable medical device and to an assembly comprising two such devices. It concerns a method and means for deploying a prosthesis within a body lumen, or guiding interventional devices into a body lumen and in particular to a stent graft and delivery device assembly, especially one having a low profile to reduce the interruption to blood flow. For example the invention may be used for introducing an expandable intraluminal prosthesis (or stent graft) in an endovascular procedure for the repair of diseased or damaged vessels.

### BACKGROUND

The deployment of intraluminal prostheses into the lumen of a patient from a remote location by the use of a deployment device or introducer, has been disclosed in a number of earlier patent specifications.

Numerous procedures have been developed that involve the percutaneous insertion of a medical device into a body lumen, such as a blood vessel or duct, of a patient's body. Such a device may be introduced into the lumen by a variety of known techniques. For example, a wire guide may be introduced into a blood vessel using the Seldinger technique. This technique involves creating a surgical opening in the vessel with a needle and inserting a wire guide into the vessel through a bore of the needle. The needle can be withdrawn, leaving the wire guide in place. A delivery device is then inserted over the wire guide and into the vessel. The delivery device may be used in conventional fashion to insert into the blood vessel a variety of medical devices, such as stents, stent grafts, catheters, cardiac leads, balloons, and the like.

For example, the delivery device may be used to deliver and deploy an expandable prosthesis, such as a stent graft, to an aneurysmal blood vessel site. A stent graft is usually formed from a tubular body of a biocompatible graft material with one or more stents mounted into or onto the tubular body to provide support therefor. The stents may be balloon expandable stents and/or self-expanding stents. The deployment of the prosthesis into the lumen of a patient from a remote location by the use of an introducer delivery and deployment device is described in, e.g., U.S. Pat. No. 7,435,253 to Hartley entitled "A Prosthesis and a Method and Means of Deploying a Prosthesis," which is incorporated herein by reference in its entirety.

Delivery devices are configured to retain a prosthesis in a delivery configuration during delivery to the desired deployment site. The delivery catheter typically includes an inner catheter/cannula spaced from an outer sheath to define a prosthesis retaining region for receiving the prosthesis. The prosthesis is loaded onto an inner cannula along a prosthesis retaining region, with an outer sheath retaining the prosthesis in the delivery configuration. After the delivery device is delivered to the desired deployment site, the prosthesis may be deployed, for example, with retraction of the outer sheath relative to the inner cannula away from the prosthesis to allow for expansion thereof. Accurate placement of an appropriately sized prosthesis should sufficiently cover the target site for treatment and the ends of the prosthesis should be engaged with healthy tissue of the body lumen.

It is desirable to load an appropriately sized prosthesis into a delivery device that is as small as possible. Besides the size of the prosthesis being a factor in the size selection of a delivery device, the shape and size of the body lumen can also be important. Thus, introducing a delivery device that is relatively smaller than the body lumen can avoid potential blockage of fluid or blood flow within the lumen, which can adversely affect the lumen and other parts of the body. Furthermore, the body lumen can be tortuous, thus making relatively smaller delivery devices easier to pass through the tortuous portions.

US 2010/0249895 A1 discloses a delivery device assembly with an introducer catheter having a structure for attaching its proximal end to a body lumen wall incorporating an inflatable balloon.

US 5 542 936 A discloses an introducer sheath the proximal end of which has fenestrations to allow blood to flow therethrough after the sheath is inserted into a body lumen.

### SUMMARY

According to a first aspect, there is provided a delivery device assembly according to claim 1.

In one embodiment, the ratio of the inner diameter of the introducer catheter to that of the engagable outer diameter of the elongate seal-engaging sleeve portion is greater than 1.2 to 1.

In one embodiment, the ratio of the inner diameter of the introducer catheter to that of the engagable outer diameter of the elongate seal-engaging sleeve portion is greater than 1.5 to 1.

In one embodiment, the assembly further comprises a dockable device slidably mounted to the seal-engaging sleeve portion, the dockable device having a seal that is adapted to sealing engage the elongate seal-engaging sleeve portion.

In one embodiment, the dockable device is movable from a first position distally adjacent to the stent graft receiving portion but spaced from the introducer to a second position on a distal end of the introducer.

In one embodiment, the dockable device includes an interlocking mechanism that reversibly interlocks the dockable device to the haemostatic device.

In one embodiment, the haemostatic device comprises at least one disk valve.

In one embodiment, the haemostatic device comprises a plurality of disk valves.

In one embodiment, the assembly further comprises a guide wire catheter, the guide wire catheter extending from the dilator to a handle at a distal end of the delivery device, the guide wire catheter slidable over a guide wire.

In one embodiment, the assembly further comprises a sleeve disposed around the guide wire, the sleeve having the stent graft receiving portion at a proximal end thereof and the elongate seal-engaging sleeve portion distal of the stent graft receiving portion.

According to a second aspect of the invention, there is provided a stent graft and delivery device assembly according to claim 2.

In one embodiment, the introducer catheter comprises an inflation fluid delivery tube, the inflation fluid delivery tube connected to the toroidal seal.

In one embodiment, the ratio of the inner diameter of the introducer catheter to that of the engagable outer diameter of the elongate seal-engaging sleeve portion is greater than 1.2 to 1.

In one embodiment, the ratio of the inner diameter of the introducer catheter to that of the engagable outer diameter of the elongate seal-engaging sleeve portion is greater than 1.5 to 1.

In one embodiment, the assembly further comprises a dockable device slidably mounted to the seal-engaging sleeve portion, the dockable device having a seal that is adapted to sealing engage the elongate seal-engaging surface.

### BRIEF DESCRIPTION OF DRAWINGS

Embodiments of the present invention will be discussed with reference to the accompanying drawings wherein:
Figure 1 is an isometric view of part of a stent graft and delivery device assembly according to an embodiment of the invention;
Figure 2 is an isometric view of an introducer, the introducer being part of the delivery device assembly shown in Figure 1;
Figure 3 is a generalised isometric view showing components of the stent graft and delivery device assembly of Figures 1 and 2;
Figure 4A is a side view showing parts of the delivery device of Figure 1;
Figures 4B and 4C are cross-sectional views through section lines B-B and C-C, as shown on Figure 4A;
Figure 5A is a diagrammatic view showing an introducer catheter of the introducer of Figure 2 inserted into a femoral artery;
Figure 5B is a close up view of the distal end of the introducer catheter shown in Figure 5A;
Figure 6 is a similar view to that of Figure 3 but shows an alternative introducer;
Figure 7 is a similar view to that of Figure 2 but shows the alternative introducer of Figure 6 in more detail;
Figures 8A and 8B show the introducer of Figures 6 and 7 in a deflated and inflated condition within a femoral artery respectively;
Figures 9A and 9B are detailed views of a portion of the alternative introducer shown in Figures 12A and 12B;
Figures 10A to 10E are diagrammatic views showing deployment of the delivery device according to the invention into an arterial system of a patient.
Figures 11 and 12 are isometric and cross-sectional views respectively of a dockable device being a component of the stent graft and the delivery device assembly shown in Figure 3;
Figure 13 is a cross-sectional view showing the delivery device of the invention passing through a haemostatic device of the invention;
Figure 14 is a similar view to that of 13 but shows the dockable device of Figures 11 and 12 docked with the haemostatic device;
Figures 15 and 16 show an alternative embodiment of the dockable device shown in Figures 11 and 12 in isometric and cross-sectional views respectively; and
Figure 17 shows the dockable device of Figures 15 and 16 docked to the haemostatic device of Figure 13.

### DESCRIPTION OF EMBODIMENTS

For the purposes understanding of the principles of the invention, reference will now be made to the embodiments illustrated in the drawings, and specific language will be used to describe the same. It is to be understood that the Figures are schematic and do not show the various components in their actual scale. In many instances, the Figures show scaled up components to assist the reader.

Throughout this specification, the term distal with respect to a portion of the aorta, a deployment device or an endograft means the end of the aorta, deployment device or endograft further away in the direction of blood flow away from the heart and the term proximal means the portion of the aorta, deployment device or end of the endograft nearer to the heart. When applied to other vessels, similar terms such as caudal and cranial should be understood.

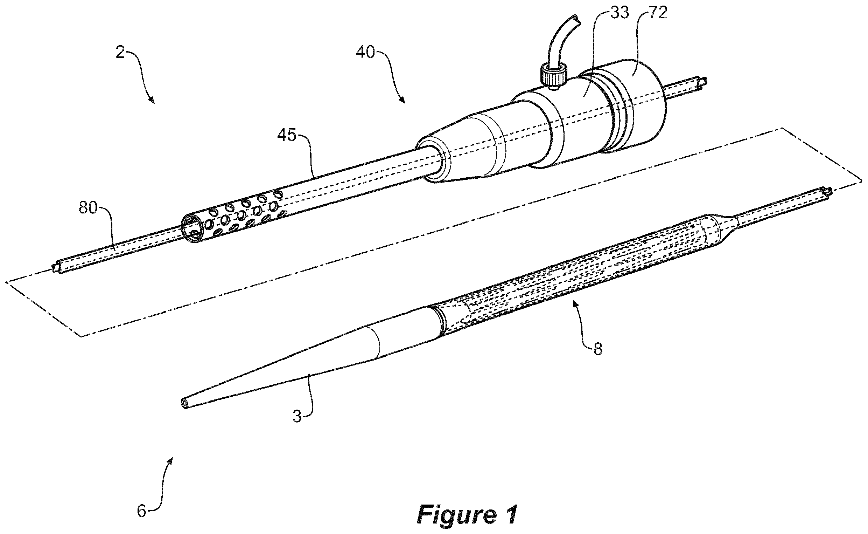

Referring now to Figure 1, there is shown a delivery device assembly for the percutaneous insertion into the artery (or other bodily lumen) of medical devices such as stents, stent grafts, catheters, cardiac leads, balloons, and the like. With the embodiment shown in Figure 1, the delivery device assembly is loaded with a stent graft.

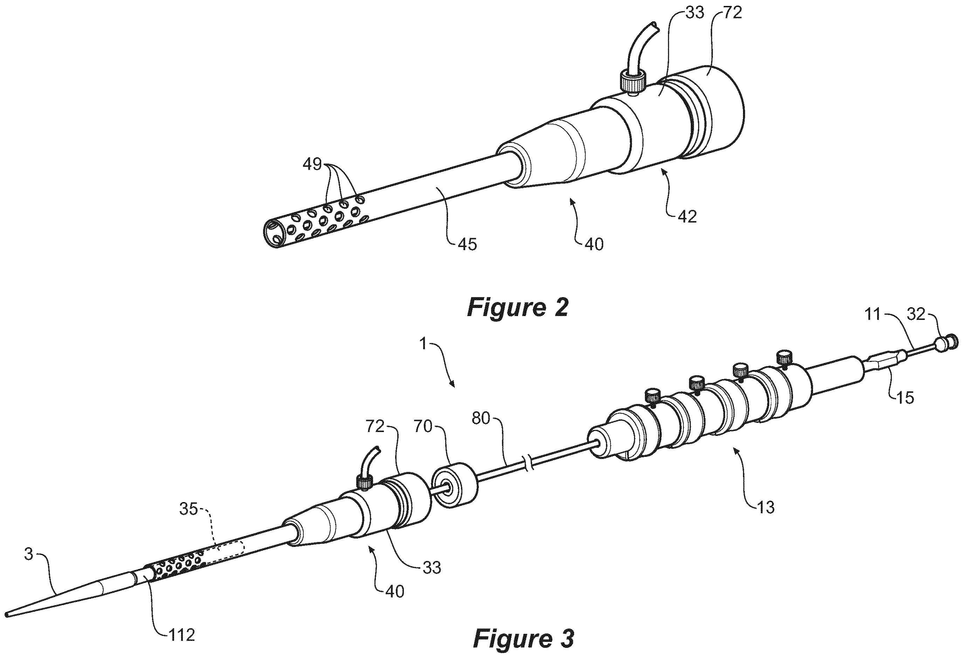

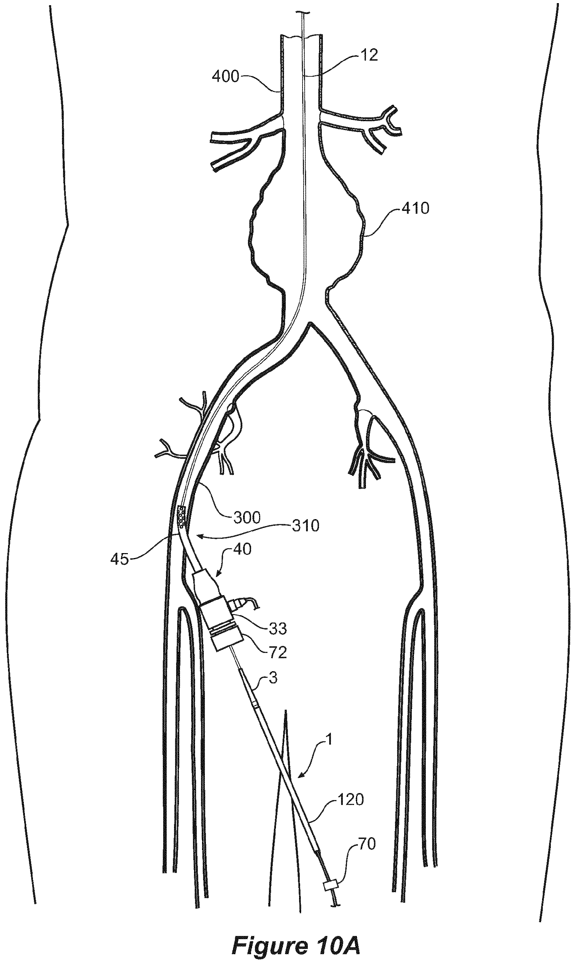

The delivery device assembly has two distinct components. The first component, an introducer 40, is shown in Figure 2. The introducer 40 has a tubular introducer catheter 45 for insertion over a guide wire into a bodily lumen such a femoral artery, for instance, as is shown in Figure 10A. The introducer 40 includes a haemostatic device 33 for controlling blood loss through the delivery device. The haemostatic device 33 is fixedly connected to the introducer catheter 45. The haemostatic device 33 comprises one or more haemostatic valves or seals. Suitable haemostatic valves include, for example, disk/disc valves, iris valves, and the like that provide a seal.

The second component of the assembly is a delivery device 2 as shown in Figure 3 passing through the introducer 40. As can be seen from Figures 1, 3 and 10A, the delivery device 2 includes a dilator 3 at a proximal end thereof. Adjacent the dilator and distal of the dilator 3 is a stent graft receiving portion 8 with a stent graft 35 mounted in the receiving portion. At a distal end of the delivery device 2 is a connection means 32 in the form of a Luer lock connector (adapted to accept a syringe for introduction of reagents).

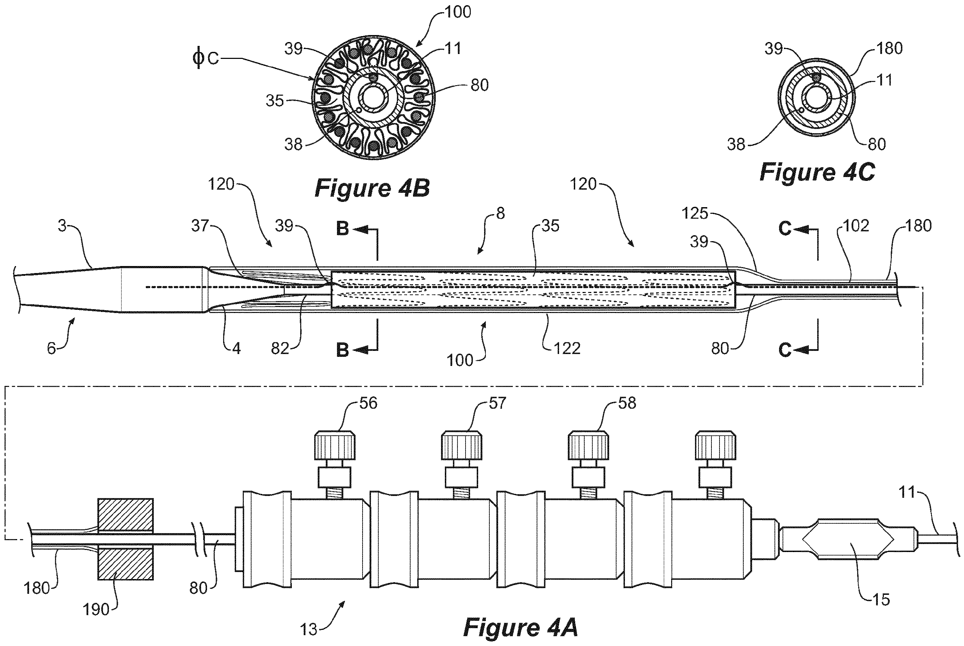

Figure 4A shows the proximal end 6 of the delivery device assembly 1, the stent graft receiving portion 8 and its associated stent graft 35 in more detail. Figure 4A shows a stent graft cover 122 in the form of a thin cover 100.

Also shown in Figure 4A is a guide wire catheter 11. The guide wire catheter 11 extends from the dilator 3 to a handle 13 at a distal end of the delivery device 2. The guide wire catheter 11 is slidable over a guide wire, such as the guide wire 12 illustrated in Figure 10A.

Still referring to Figure 4A, the cover 122 transitions at transition portion 125 into an elongate seal-engaging sleeve portion 180 that extends right though the introducer 40, and its haemostatic device 33, and out of its distal end external to the patient's body. A puller 190 is provided so as to actuate the removal of the cover 122. This seal-engaging sleeve portion 180 engages a seal within a haemostatic device 33 that forms part of the introducer 40 shown in Figure 2. More specifically, the engagable maximum outer diameter of the seal-engaging sleeve portion 180 is engaged by the disk valves or seals 33' of the haemostatic device 33.

Turning now to Figure 4B, which is a cross-sectional view through section lines B-B on Figure 4A, the stent graft 35 can be seen disposed between the cover 100 and the guide wire
catheter 11. A guide wire catheter sleeve 80 between the guide wire catheter 11 and the stent graft 35 may also be provided, as is shown in Figures 4A-4C.

Turning now to Figure 4C, inboard of the sleeve 80 is a space formed between the inside of the sleeve 80 and the outside of the guide wire catheter 11. This space accommodates a stent graft ends release wire 39 (or two stent graft end release wires - one for a proximal end and one for a distal end) and a stent graft compression release wire 38. The stent graft ends release wire 39 exits the sleeve 80 in the two positions shown in Figure 4A so as to releasably retain the proximal and distal ends of the stent graft 80, as is known in the art. The stent graft compression release wire 38 also exits the sleeve 80 so as to selectively retain and release the stent graft using diameter releasing ties or other methods known in the art.

A cross-sectional view of Figure 4C, taken through section lines C-C, show that the overall diameter of the delivery device 2 reduces in a direction distal of the stent graft receiving portion 8 of the delivery device 2. The reduced overall diameter assists in minimising the interruption to blood flow that may otherwise be caused by larger diameter delivery devices.

The stent graft cover 100 of the invention may take many forms. With the form shown in Figures 1, 3, 4A, 4B and 4C, the cover 100 is part of an outermost sheath 120. The outermost sheath 120 has a cover 100 that transitions an elongate seal-engaging sleeve portion 180 through a transition portion 125, as is most clearly shown in Figure 4A. The outermost sheath 120 may be made from various suitable materials. It may be made from a material that holds its general shape to the shape shown in the figures. Alternatively, it may be made from a stretchable, resilient material that stretches over the loaded stent graft 35 in the cover region 100.

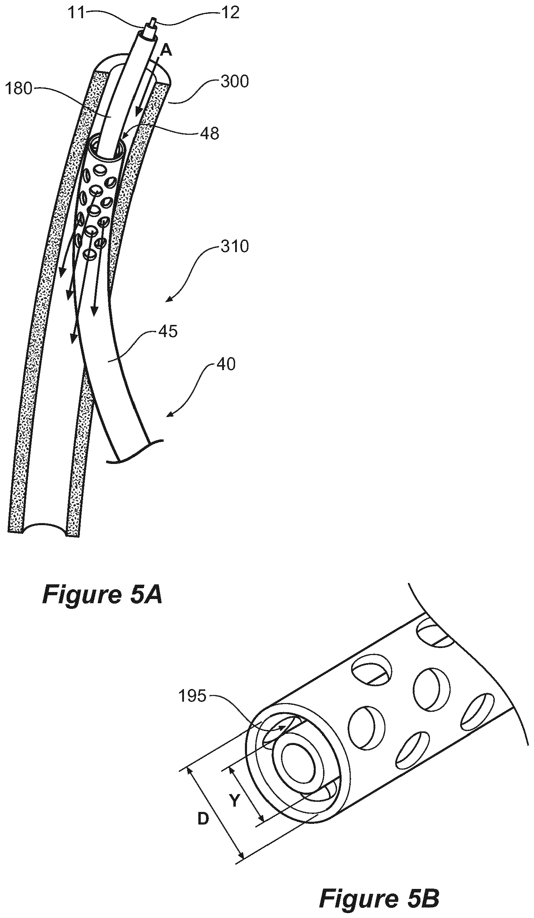

The introducer 40 disposed around the sleeve 80 and valve-engaging sleeve portion 180 is more clearly seen in Figure 5A. The tubular introducer catheter 45 of the introducer 40 has an inner diameter D, the inner diameter D greater than the engagable outer diameter Y of the elongate seal-engaging sleeve portion 180, as is illustrated in Figure 5B. This diameter difference forms an annular lumen 195 through which blood may flow.

The elongate seal-engaging sleeve portion 180 has an engagable maximum outer diameter Y that is less than the maximum outer diameter C of the cover 100, as is illustrated in Figure 4B read together with Figures 5A and 5B.

The ratio of the inner diameter D of the introducer catheter 45 to that of the outer diameter Y of the elongate seal-engaging sleeve portion 180 is greater than 1.2 to 1. In fact, with the embodiment shown in the Figures, the ratio is greater than 1.5 to 1. As this ratio increases, the extent to which the delivery device 2 impedes the blood flow is reduced.

As can be seen in Figure 5A, the tubular introducer catheter 45 of the introducer 40 takes up a considerable portion of the femoral artery 300. With conventional delivery devices, the tubular introducer catheter 45 extends a longer distance up the femoral artery 300 and into the aorta. This significantly reduces blood flow through the femoral artery 300 at and proximally of the introduction zone 310. With embodiments of the present invention, blood flow in the direction indicated by arrow A on Figure 5A is less interrupted. This is because the blood may flow into the mouth 48 of the introducer catheter 45 and flow along the aforementioned annular lumen 195 and out through apertures 49.

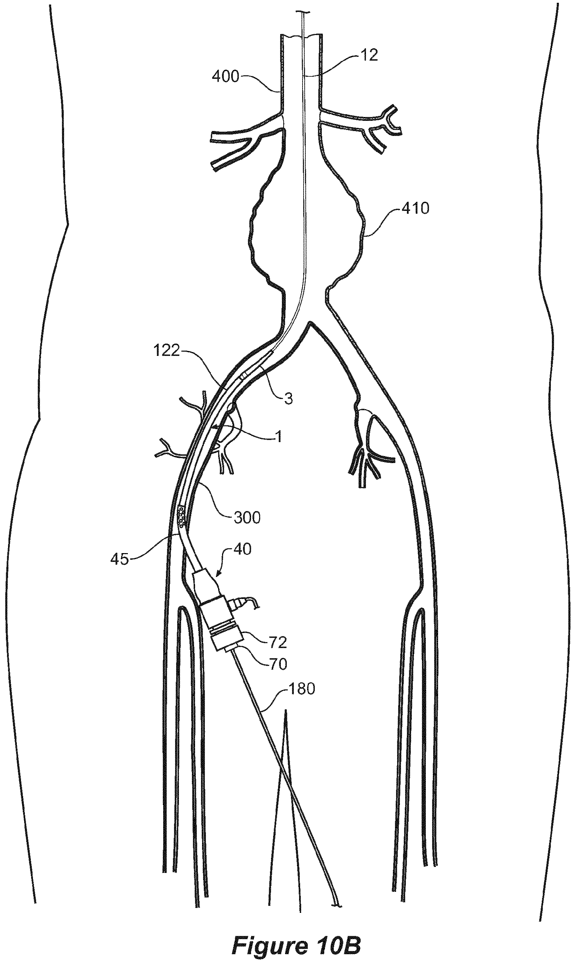

Referring again to Figure 3, a dockable device 70 and a dock 72 are shown. The thickest part of the delivery device 2 is the nose cone dilator 3 and the adjacent stent graft cover portion 122 which need to pass through the haemostatic device 33 of the introducer 40 as occurs between the positions shown in Figures 10A and 10B. The dockable device 70 assists with providing a more effective seal around the smaller diameter elongate seal-engaging sleeve portion 180.

The dockable device 70 has a tight seal provided by a seal 78, in the form of an O-ring for instance, which is sized to accommodate the elongate seal-engaging sleeve portion 180. Other appropriate types of seals instead of O-ring-type seals may also be used. The dockable device 70 docks into the dock 72.

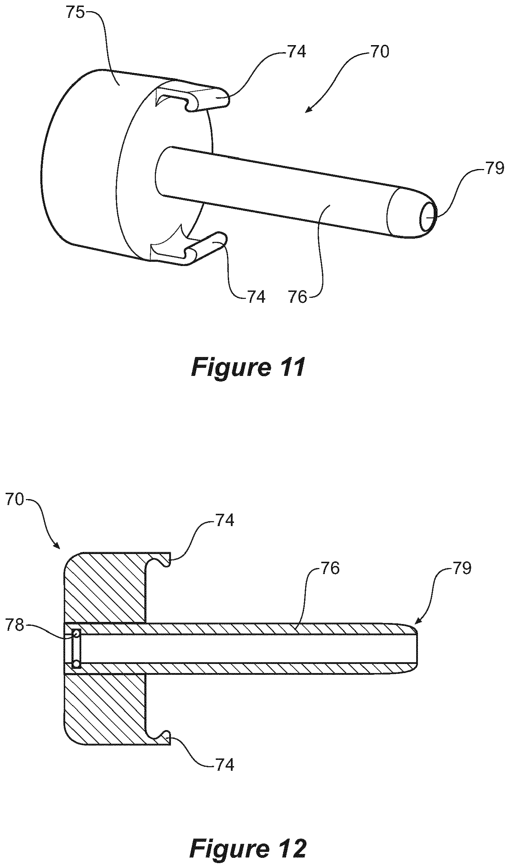

Referring to Figures 11, 12, 13 and 14, the dockable device 70 will be described in more detail. The dockable device 70 is slidably mounted to the seal-engaging sleeve portion 180. An annular seal 78, as is most clearly shown in Figure 12, is provided to seal against the elongate portion 180. More specifically, the engagable maximum outer diameter Y of the seal-engaging sleeve portion 180 is engaged by the annular seal 78.

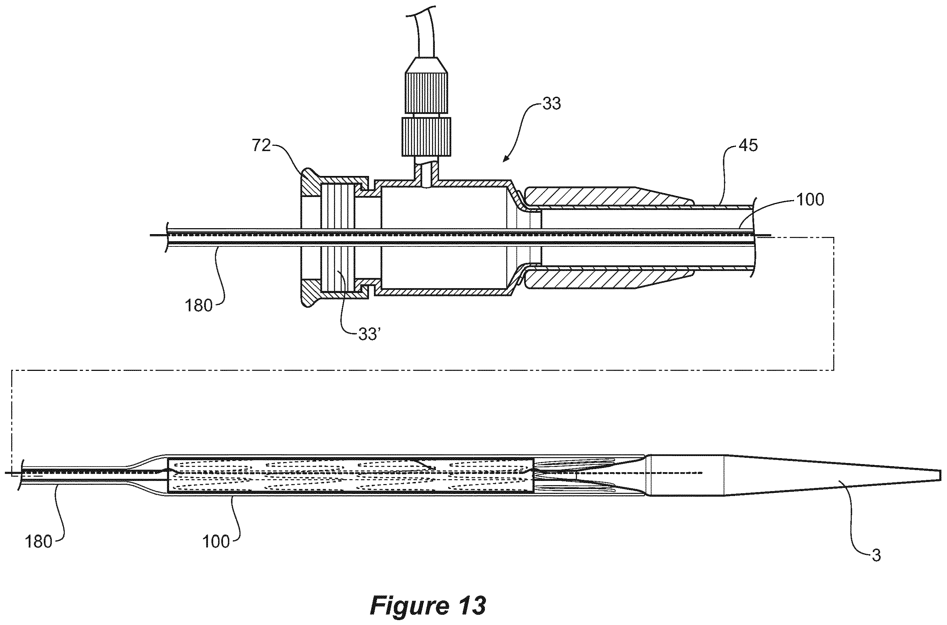

The dockable device 70 includes a body 75 and a sleeve 76. Interlocking mechanisms 74 are provided to reversibly interlock the dockable device 70 to the haemostatic device 33 at dock 72 (as illustrated in Figure 13).

The aforementioned dockable device 70 is similar to the dockable device described in the applicant's earlier US Patent No. 8,419,783.

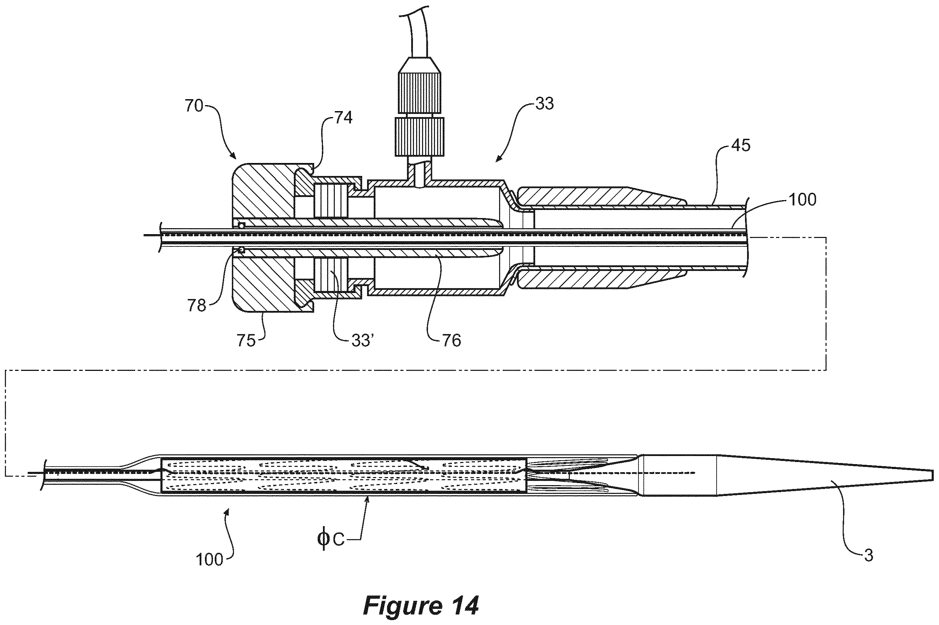

With the dockable device 70 shown in Figures 11, 12 and 14, the sleeve 76 has an outer diameter that is similar to or the same as the maximum outer diameter D of the stent graft cover 100. This helps ensure a good seal through the disc valves 33', as is illustrated in Figure 14.

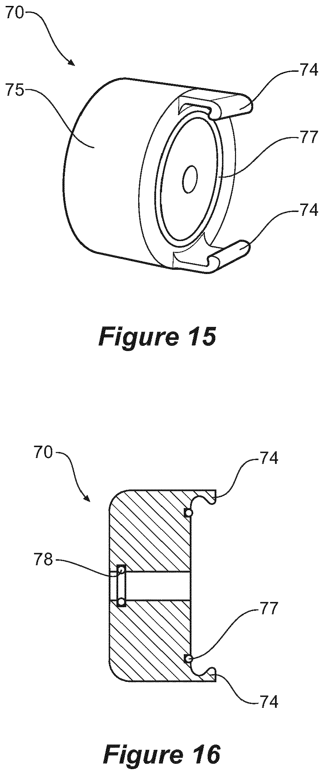

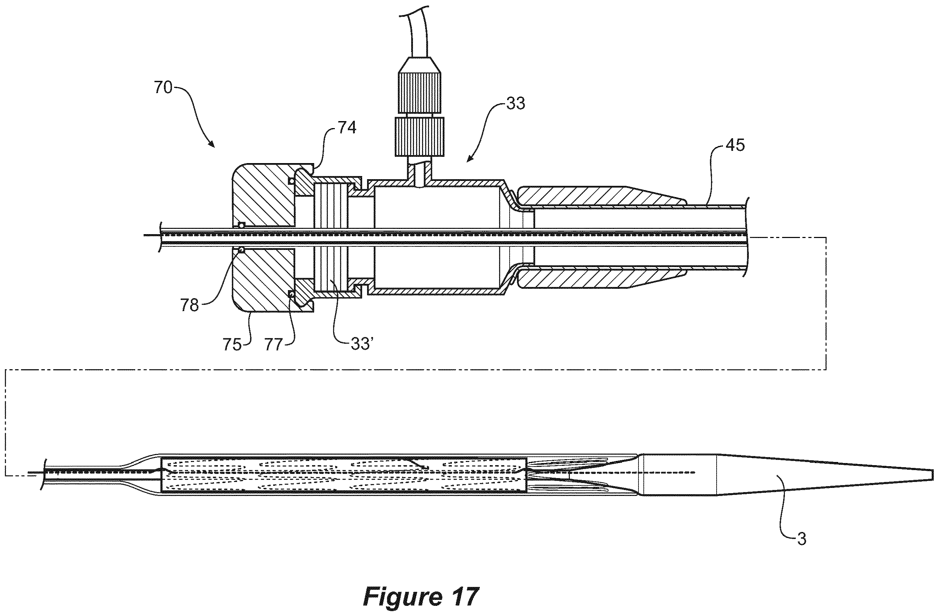

An alternative dockable device 70 is shown in Figures 15, 16 and 17. With this version, there is no sleeve 76. An O-ring seal 77 may be provided so as to seal the body 75 against the dock 72. This O-ring seal 77 is not essential and may be deleted in some embodiments as the disc valves 33'provide sealing, However, the additional sealing may be helpful in some applications.

The guide wire catheter 11 may comprise any suitable biocompatible material or materials including metal or plastic. Suitable materials include, but are not limited to aluminum, nitinol, nylon, polypropylene, and polyethylene. The guide wire catheter sleeve 80 preferably comprises a flexible material that is able to bend and flex to negotiate complex and tortuous inner body lumina. The sleeve 80 may comprise a biocompatible plastic such as PTFE, PEEK, polyethylene, nylon, or the like.

The guide wire catheter sleeve 80, or the guide wire catheter sleeve 80 in combination with the guide wire catheter 11, are constructed to have sufficient longitudinal column strength to ensure adequate pushing force manipulation during delivery and deployment of the stent graft 35.

Figures 10A-10E illustrates an example of use of the delivery device 2 deploying a medical device, such as the stent graft 35, into an artery.

The delivery device 2 may be used to percutaneously insert into the artery (or other bodily lumen) a variety of medical devices, such as stents, stent grafts, catheters, cardiac leads, balloons, prostheses, and the like.

A wire guide may be introduced into an artery using the Seldinger technique. This technique involves creating a surgical opening in artery with a needle and inserting a wire guide into the artery through a bore of the needle. The needle can be withdrawn, leaving the wire guide in place.

In Figure 10A, the tubular introducer catheter 45 of the introducer 40 is inserted over a guide wire 12 into the femoral artery 300 at an introduction zone 310. At this point, the nose cone 3 of the delivery device 2 is distal of the introducer 40. Even further distally is the dockable device 70.

From the position shown in Figure 10A, the delivery device 2 is advanced over the guide wire 12 to the position shown in Figure 10B. As can be seen in Figure 10B, the thickest part of the delivery device 2 has passed through the haemostatic device 33 of the introducer 40. In this position, the elongate seal-engaging sleeve portion 180 is engaged with the disk seal 33 within the haemostatic device 33. The dockable device 70, as for instance shown in Figures 15 and 16, assists with providing a more effective seal around the smaller diameter elongate seal-engaging sleeve portion 180, as has been described above.

The docking of the dockable device to the dock 72 may be achieved by means such as a common bayonet or a sleeve with locking screw, or screw thread, or push clip, or any other appropriate means. Figures 15 and 16 show a clipable interlocking mechanism 74.

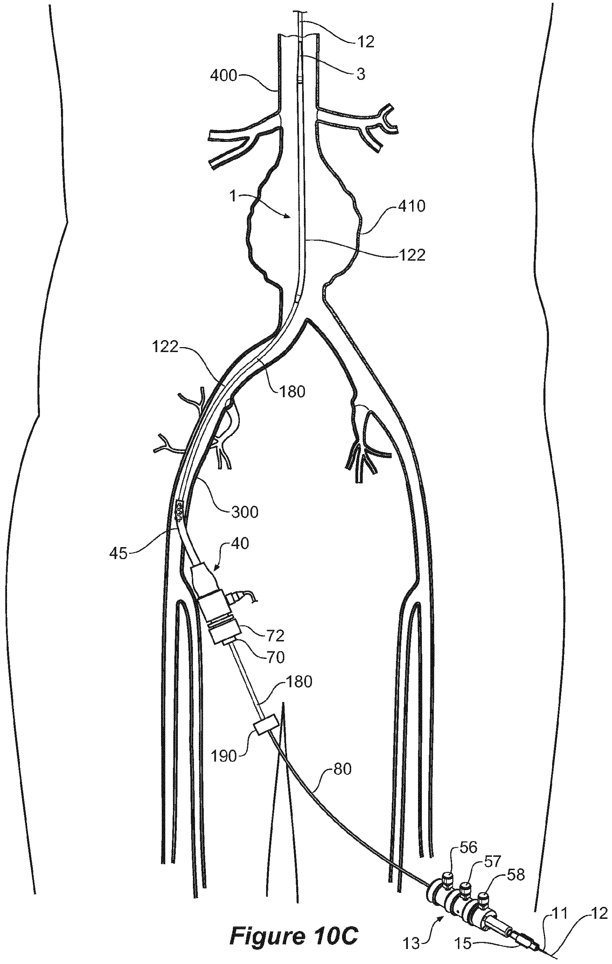

Turning now to Figure 10C, the delivery device 2 has been advanced proximally to its approximate deployment position within aneurysmal portion 410 of the aorta 400. In this Figure, the handle 13 is now visible. The next step in the deployment procedure is the retraction of the stent graft cover portion 122 of the outermost sheath 120. This step will vary depending on the embodiment of the invention being used.

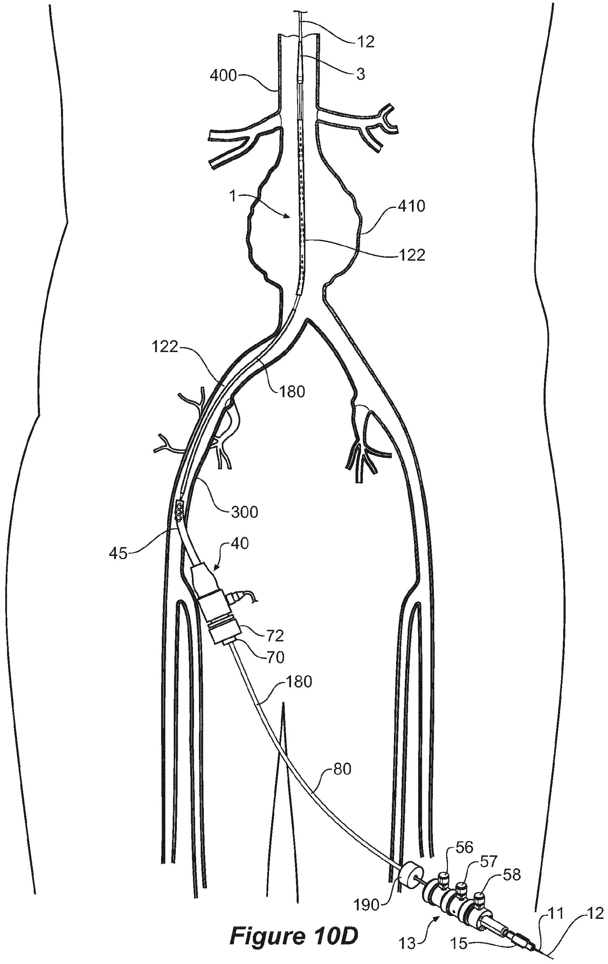

With the embodiment of the invention shown in Figures 4A-4C, sliding the puller 190 at the distal end of the outermost sheath 120 distally to the position shown in Figure 10D retracts the cover portion 122 of the outermost sheath 120. In this position, it can be seen that the stent graft cover portion 122 of the outermost sheath 120 is now distal of the stent graft 35.

With the stent graft 35 now uncovered, the diameter reducing ties locking screw 58 is removed and the stent graft compression release wire 38 is withdrawn. This allows the stents of the stent graft 35 to expand.

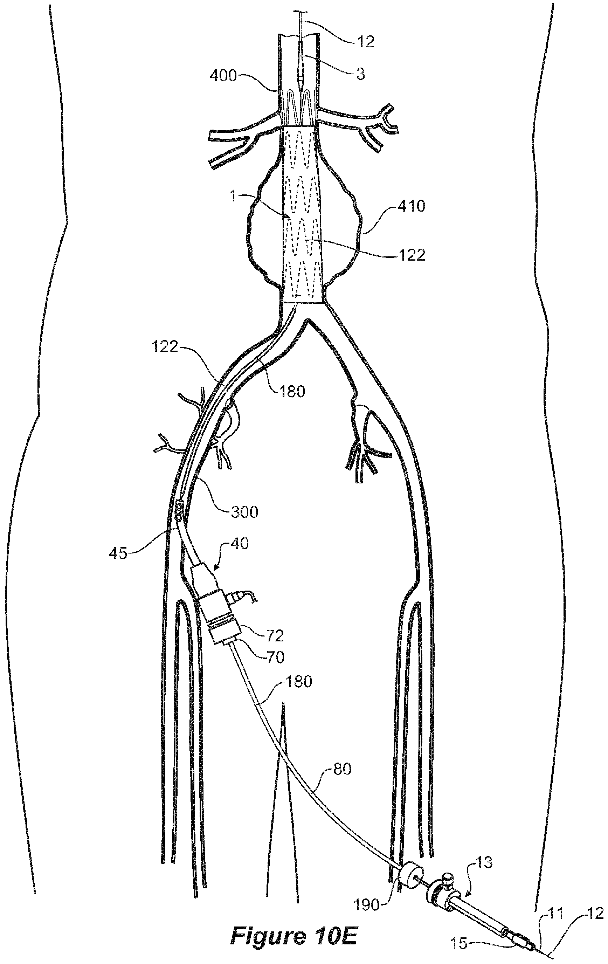

After the stent graft 35 has expanded, the locking screws 56 and 57 are released and the stent graft ends release wires are removed. This frees the proximal and distal ends of the stent graft 35 from the delivery device 2, allowing the stent graft 35 to adopt its final position, as shown in Figure 10E.

In other embodiments of the invention, a single stent graft end release wire 39 may be used with a single locking screw 37. With such an embodiment, the stent graft end release wire 39 (such as is shown in Figure 4A for instance), can be progressively withdrawn so as to first release the proximal end of the stent graft 35 and subsequently the distal end of the stent graft 35.

After deployment of the stent graft 35, the delivery device 2 can be progressively removed. The introducer 40 of the delivery device 2 may remain in its position shown in Figures 10A to 10E, so as to permit the subsequent passage of other medical devices therethrough into the arterial system.

The delivery device 2 as described above includes an introducer 40 having a tubular introducer catheter 45 with a plurality of apertures 49 provided to facilitate blood flow past the delivery device 2.

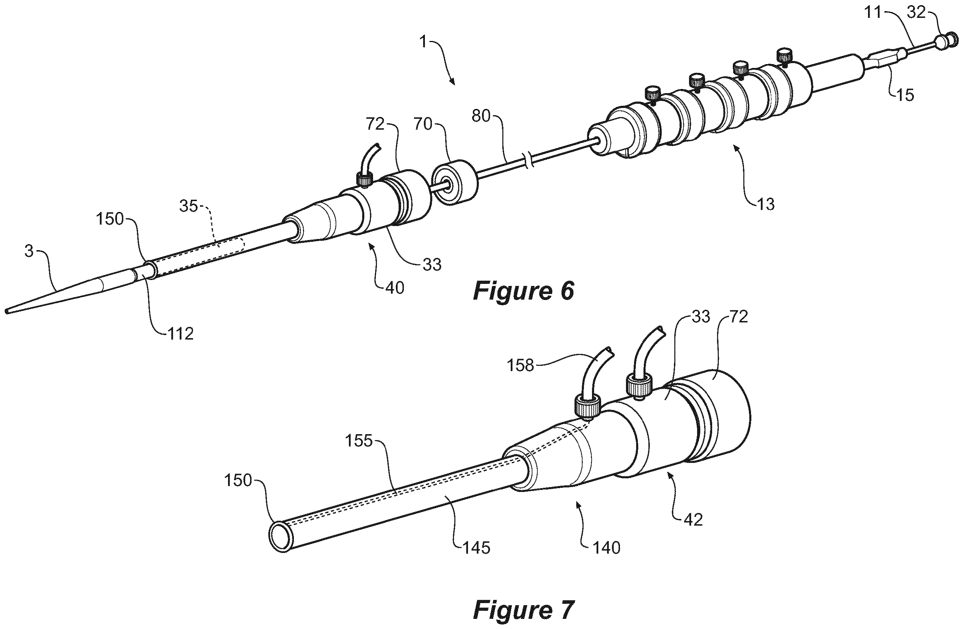

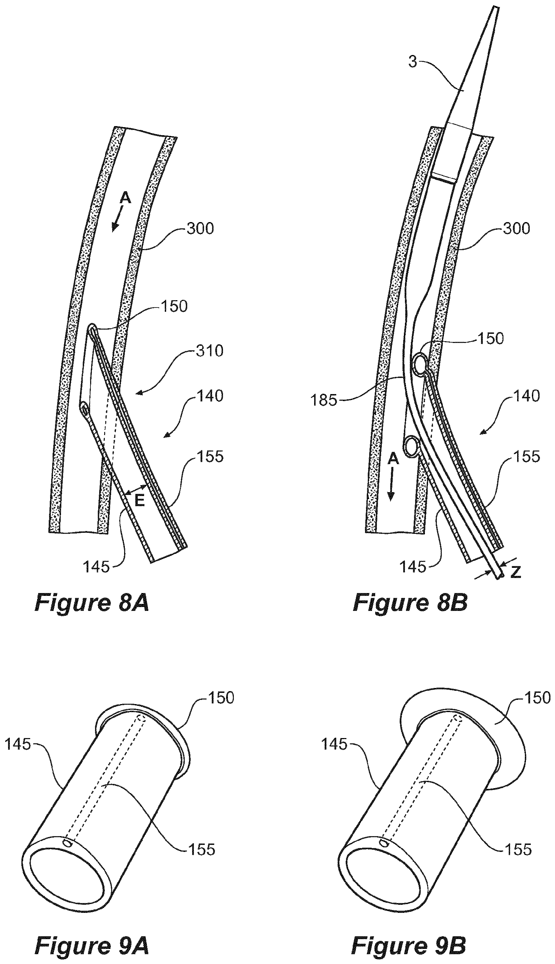

An alternative introducer 140 is shown in Figures 7, 8A, 8B, 9A and 9B. The alternative introducer 140 has the same distal end with a haemostatic device 33 and dockable device 70 but has an alternative tubular introducer catheter 145. The alternative introducer catheter 145 terminates with an inflatable seal 150. Figures 8A and 9A show the inflatable seal 150 in a deflated condition.

The introducer 140 is inserted over a guide wire 12 into the femoral artery 300 in a similar way to the introducer 140, as described above. This insertion is done with the seal 150 deflated. Once in the position illustrated in Figure 8A, the inflatable seal 150 is inflated by delivery of a fluid (for instance, sterile saline) via a delivery line 155. The delivery line 155, as shown in Figures 9A and 9B, may be integrated into the introducer catheter 145 or may be a separate line. An inlet port 158 such as is shown in Figure 7 may be provided to facilitate fluid delivery.

Once the inflatable seal 150 has been inflated, the introducer 140 may be slightly retracted from the position shown in Figure 8A to the position shown in Figure 8B. The inner diameter E is greater than the outer diameter Z of the valve-engaging external surface 80. This, in combination with the introducer 140 shape and positioning facilitates blood flow in the direction of Arrow A past the insertion point of the introducer 140.

Throughout the specification and the claims that follow, unless the context requires otherwise, the words "comprise" and "include" and variations such as "comprising" and "including" will be understood to imply the inclusion of a stated integer or group of integers, but not the exclusion of any other integer or group of integers.

The reference to any prior art in this specification is not, and should not be taken as, an acknowledgement of any form of suggestion that such prior art forms part of the common general knowledge.

It will be appreciated by those skilled in the art that the invention is not restricted in its use to the particular application described. Neither is the present invention restricted in its preferred embodiment with regard to the particular elements and/or features described or depicted herein. It will be appreciated that the invention is not limited to the embodiment or embodiments disclosed, but is capable of numerous rearrangements, modifications and substitutions without departing from the scope of the invention as set forth and defined by the following claims.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A delivery device assembly (2) comprising a portion for receiving an insertable medical device (35), a cover (100) mounted around said portion, and an introducer (40) with an introducer catheter (45) arranged to be disposed around the cover and having a proximal end in the form of an insertion portion which has a structure which allows flow past the insertion portion when inserted in a lumen, the introducer having a hollow body and a haemostatic device (33), the introducer catheter extending proximally from the hollow body, **characterised in that** the device further comprises an elongate seal-engaging sleeve portion (180) disposed distally of the medical device receiving portion, the cover transitions at a transition point (125) into such elongate seal-sealing sleeve portion, the seal-engaging sleeve portion having an engagable outer diameter, the haemostatic device comprises at least one seal engageable by the engageable outer diameter of the elongate seal-engaging sleeve portion, **in that** the introducer catheter (45) has an inner diameter (D) greater than the engagable outer diameter (Y) of the elongate seal-engaging sleeve portion (180),
and **in that**, to allow said flow, the introducer catheter (45) comprises an external tubular wall, the wall having a plurality of holes (49), the holes being arranged to facilitate blood flow from upstream of the introduction zone into a vascular system to downstream of the introduction zone..

2. A delivery device assembly (2) comprising a portion for receiving an insertable medical device (35), a cover (100) mounted around said portion, and an introducer (140) with an introducer catheter (145) arranged to be disposed around the cover and having a proximal end in the form of an insertion portion which has a structure which allows flow past the insertion portion when inserted in a lumen, the introducer having a hollow body and a haemostatic device (33), the introducer catheter extending proximally from the hollow body, **characterised in that** the device further comprises an elongate seal-engaging sleeve portion (180) disposed distally of the medical device receiving portion, the cover transitions at a transition point (125) into such elongate seal-sealing sleeve portion, the seal-engaging sleeve portion having an engagable outer diameter, the haemostatic device comprises at least one seal engageable by the engageable outer diameter of the elongate seal-engaging sleeve portion, **in that** the introducer catheter (145) has an inner diameter (D) greater than the engagable outer diameter (Y) of the elongate seal-engaging sleeve portion (180), and **in that**, to allow said flow, the introducer catheter (145) comprises an external tubular wall and a toroidal seal (150) at a proximal end thereof, wherein the toroidal seal (150) is expandable from a deflated condition to an inflated condition.

3. A delivery device assembly according to claim 2 wherein the introducer catheter (145) comprises an inflation fluid delivery tube (155), the inflation fluid delivery tube being connected to the toroidal seal.

4. A delivery device assembly according to any preceding claim further comprising a dilator (3) disposed proximally of the medical device receiving portion, and
the elongate seal-engaging sleeve portion having a maximum outer diameter less than a maximum outer diameter of the cover.

5. A delivery device assesmbly according to any preceding claim wherein the ratio of the inner diameter (D) of the introducer catheter (45, 145) to that of the engagable outer diameter (Y) of the elongate seal-engaging sleeve portion (180) is greater than 1.2 to 1 and in particular greater than 1.5 to 1.

6. A delivery device assembly according to any preceding claim comprising a dockable device (70) slidably mounted to the seal-engaging sleeve portion, the dockable device having a seal (78) that is adapted to sealing engage the elongate seal-engaging sleeve portion (180).

7. A delivery assembly device according to claim 6 wherein the dockable device (70) is movable from a first position distally adjacent to the medical device receiving portion but spaced from the introducer (40) to a second position on a distal end of the introducer.

8. A delivery device assembly according to claim 6 or 7 wherein the dockable device (70) includes an interlocking mechanism (74) that reversibly interlocks the dockable device to the haemostatic device (33).

9. A delivery device assembly according to any preceding claim comprising a guide wire catheter (11), the guide wire catheter extending from a dilator (3) to a handle at a distal end of the delivery device, the guide wire catheter being slidable over a guide wire (12).

10. A delivery device assembly according to claim 9 comprising a sleeve (80) disposed around the guide wire, the sleeve having the medical device receiving portion at a proximal end thereof and the elongate seal-engaging sleeve portion being distal of the medical device receiving portion.

## Patentansprüche

1. Freisetzungsvorrichtungsanordnung (2), umfassend einen Abschnitt zur Aufnahme einer einführbaren medizinischen Vorrichtung (35), eine Abdeckung (100), die um den Abschnitt herum angebracht ist, und ein Einführungsinstrument (40) mit einem Einführungskatheter (45), der zur Anordnung um die Abdeckung ausgelegt ist und ein proximales Ende in Form eines Einführungsabschnitts aufweist, dessen Struktur Durchfluss am Einführungsabschnitt vorbei gestattet, wenn er in ein Lumen eingeführt ist, wobei das Einführungsinstrument einen hohlen Körper und eine hämostatische Vorrichtung (33) aufweist, wobei sich der Einführungskatheter proximal vom hohlen Körper erstreckt, **dadurch gekennzeichnet, dass** die Vorrichtung ferner einen langgestreckten Dichtungseingriffshülsenabschnitt (180) umfasst, der distal vom Aufnahmeabschnitt der medizinischen Vorrichtung angeordnet ist, wobei die Abdeckung an einem Übergangspunkt (125) in einen solchen langgestreckten Dichtungseingriffshülsenabschnitt übergeht, wobei der Dichtungseingriffshülsenabschnitt einen in Eingriff bringbaren Außendurchmesser aufweist, wobei die hämostatische Vorrichtung mindestens eine Dichtung umfasst, die von dem in Eingriff bringbaren Außendurchmesser des langgestreckten Dichtungseingriffshülsenabschnitts in Eingriff genommen werden kann, dass der Einführungskatheter (45) einen Innendurchmesser (D) aufweist, der größer als der in Eingriff bringbare Außendurchmesser (Y) des langgestreckten Dichtungseingriffshülsenabschnitts (180) ist, und dass der Einführungskatheter (45) zum Gestatten des Durchflusses eine röhrenförmige Außenwand umfasst, wobei die Wand eine Vielzahl von Löchern (49) aufweist, wobei die Löcher zur Erleichterung des Blutflusses von stromaufwärts von der Einführungszone in ein Gefäßsystem nach stromabwärts von der Einführungszone angeordnet sind.

2. Freisetzungsvorrichtungsanordnung (2), umfassend einen Abschnitt zur Aufnahme einer einführbaren medizinischen Vorrichtung (35), eine Abdeckung (100), die um den Abschnitt herum angebracht ist, und ein Einführungsinstrument (140) mit einem Einführungskatheter (145), der zur Anordnung um die Abdeckung ausgelegt ist und ein proximales Ende in Form eines Einführungsabschnitts aufweist, dessen Struktur Durchfluss am Einführungsabschnitt vorbei gestattet, wenn er in ein Lumen eingeführt ist, wobei das Einführungsinstrument einen hohlen Körper und eine hämostatische Vorrichtung (33) aufweist, wobei sich der Einführungskatheter proximal vom hohlen Körper erstreckt, **dadurch gekennzeichnet, dass** die Vorrichtung ferner einen langgestreckten Dichtungseingriffshülsenabschnitt (180) umfasst, der distal vom Aufnahmeabschnitt der medizinischen Vorrichtung angeordnet ist, wobei die Abdeckung an einem Übergangspunkt (125) in einen solchen langgestreckten Dichtungseingriffshülsenabschnitt übergeht, wobei der Dichtungseingriffshülsenabschnitt einen in Eingriff bringbaren Außendurchmesser aufweist, wobei die hämostatische Vorrichtung mindestens eine Dichtung umfasst, die von dem in Eingriff bringbaren Außendurchmesser des langgestreckten Dichtungseingriffshülsenabschnitts in Eingriff genommen werden kann, dass der Einführungskatheter (145) einen Innendurchmesser (D) aufweist, der größer als der in Eingriff bringbare Außendurchmesser (Y) des langgestreckten Dichtungseingriffshülsenabschnitts (180) ist, und dass der Einführungskatheter (145) zum Gestatten des Durchflusses eine röhrenförmige Außenwand und eine Toroid-Dichtung (150) an einem proximalen Ende davon umfasst, wobei die Toroid-Dichtung (150) aus einem entleerten Zustand in einen aufgeblasenen Zustand expandierbar ist.

3. Freisetzungsvorrichtungsanordnung nach Anspruch 2, wobei der Einführungskatheter (145) einen Schlauch (155) zur Abgabe von Inflationsflüssigkeit umfasst, wobei der Schlauch zur Abgabe von Inflationsflüssigkeit mit der Toroid-Dichtung verbunden ist.

4. Freisetzungsvorrichtungsanordnung nach einem der vorhergehenden Ansprüche, ferner umfassend einen Dilatator (3), der proximal vom Aufnahmeabschnitt der medizinischen Vorrichtung angeordnet ist, und
wobei der langgestreckte Dichtungseingriffsabschnitt einen maximalen Außendurchmesser aufweist, der kleiner als ein maximaler Außendurchmesser der Abdeckung ist.

5. Freisetzungsvorrichtungsanordnung nach einem der vorhergehenden Ansprüche, wobei das Verhältnis des Innendurchmessers (D) des Einführungskatheters (45, 145) zu dem des in Eingriff bringbaren Außendurchmessers (Y) des langgestreckten Dichtungseingriffshülsenabschnitts (180) größer als 1,2 zu 1 und insbesondere größer als 1,5 zu 1 ist.

6. Freisetzungsvorrichtungsanordnung nach einem der vorhergehenden Ansprüche, umfassend eine andockbare Vorrichtung (70), die verschiebbar am Dichtungseingriffshülsenabschnitt angebracht ist, wobei die andockbare Vorrichtung eine Dichtung (78) aufweist, die für einen dichtenden Eingriff mit dem langgestreckten Dichtungseingriffshülsenabschnitt (180) ausgelegt ist.

7. Freisetzungsvorrichtungsanordnung nach Anspruch 6, wobei die andockbare Vorrichtung (70) aus einer ersten Position distal neben dem Aufnahmeabschnitt der medizinischen Vorrichtung, aber im Abstand vom Einführungsinstrument (40), in eine zweite Position an einem distalen Ende des Einführinstruments bewegbar ist.

8. Freisetzungsvorrichtungsanordnung nach Anspruch 6 oder 7, wobei die andockbare Vorrichtung (70) einen Verriegelungsmechanismus (74) aufweist, der die andockbare Vorrichtung reversibel an der hämostatischen Vorrichtung (33) verriegelt.

9. Freisetzungsvorrichtungsanordnung nach einem der vorhergehenden Ansprüche, umfassend einen Führungsdrahtkatheter (11), wobei sich der Führungsdrahtkatheter von einem Dilatator (3) bis zu einem Griff an einem distalen Ende der Freisetzungsvorrichtung erstreckt, wobei der Führungsdrahtkatheter über einem Führungsdraht (12) verschiebbar ist.

10. Freisetzungsvorrichtungsanordnung nach Anspruch 9, umfassend eine Hülse (80), die um den Führungsdraht angeordnet ist, wobei die Hülse den Aufnahmeabschnitt der medizinischen Vorrichtung an einem proximalen Ende davon aufweist und sich der langgestreckte Dichtungseingriffshülsenabschnitt distal von dem Aufnahmeabschnitt der medizinischen Vorrichtung befindet.

## Revendications

1. Ensemble dispositif de mise en place (2) comprenant une partie pour recevoir un dispositif médical (35) insérable, un couvercle (100) monté autour de ladite partie, et un introducteur (40) avec un cathéter (45) d'introducteur agencé pour être disposé autour du couvercle et comportant une extrémité proximale sous la forme d'une partie d'insertion qui présente une structure qui permet un écoulement au-delà de la partie d'insertion quand il est introduit dans une lumière, l'introducteur comportant un corps creux et un dispositif hémostatique (33), le cathéter d'introducteur s'étendant proximalement depuis le corps creux,
**caractérisé en ce que** le dispositif comprend en outre une partie de manchon (180) allongée, en prise avec un joint, disposée distalement par rapport à la partie de réception d'un dispositif médical, le couvercle se transforme en un point de transition (125) en une telle partie de manchon allongée, en prise avec un joint, la partie de manchon en prise avec un joint ayant un diamètre extérieur pouvant s'enclencher, le dispositif hémostatique comprend au moins un joint d'étanchéité pouvant entrer en prise avec le diamètre extérieur pouvant s'enclencher de la partie de manchon allongée, en prise avec un joint,
**en ce que** le cathéter (45) d'introducteur a un diamètre intérieur (D) supérieur au diamètre extérieur (Y) pouvant s'enclencher de la partie de manchon (180) allongée, en prise avec un joint, et
**en ce que**, pour permettre ledit écoulement, le cathéter (45) d'introducteur comprend une paroi externe tubulaire, la paroi comportant une pluralité de trous (49), les trous étant disposés pour faciliter le flux sanguin de l'amont de la zone d'introduction dans un système vasculaire à l'aval de la zone d'introduction.

2. Ensemble dispositif de mise en place (2) comprenant une partie pour recevoir un dispositif médical (35) insérable, un couvercle (100) monté autour de ladite partie, et un introducteur (140) avec un cathéter (145) d'introducteur agencé pour être disposé autour du couvercle et comportant une extrémité proximale sous la forme d'une partie d'insertion qui présente une structure qui permet un écoulement au-delà de la partie d'insertion quand il est introduit dans une lumière, l'introducteur comportant un corps creux et un dispositif hémostatique (33), le cathéter d'introducteur s'étendant proximalement depuis le corps creux,
**caractérisé en ce que** le dispositif comprend en outre une partie de manchon (180) allongée, en prise avec un joint, disposée distalement par rapport à la partie de réception d'un dispositif médical, le couvercle se transforme en un point de transition (125) en une telle partie de manchon allongée, en prise avec un joint, la partie de manchon en prise avec un joint ayant un diamètre extérieur pouvant s'enclencher, le dispositif hémostatique comprend au moins un joint d'étanchéité pouvant entrer en prise avec le diamètre extérieur pouvant s'enclencher de la partie de manchon allongée, en prise avec un joint,
**en ce que** le cathéter (145) d'introducteur a un diamètre intérieur (D) supérieur au diamètre extérieur (Y) pouvant s'enclencher de la partie de manchon (180) allongée, en prise avec un joint, et
**en ce que**, pour permettre ledit écoulement, le cathéter (145) d'introducteur comprend une paroi externe tubulaire et un joint d'étanchéité (150) toroïdal à son extrémité proximale,
**en ce que** le joint d'étanchéité (150) toroïdal peut se dilater d'un état dégonflé à un état gonflé.

3. Ensemble dispositif de mise en place selon la revendication 2, dans lequel le cathéter (145) d'introducteur comprend un tube (155) de débit de fluide de gonflage, le tube de débit de fluide de gonflage étant raccordé au joint d'étanchéité toroïdal.

4. Ensemble dispositif de mise en place selon l'une quelconque des revendications précédentes, comprenant en outre un dilatateur (3) disposé proximalement par rapport à la partie de réception d'un dispositif médical, et la partie de manchon allongée, en prise avec un joint ayant un diamètre extérieur maximal inférieur au diamètre extérieur maximal du couvercle.

5. Ensemble dispositif de mise en place selon l'une quelconque des revendications précédentes, dans lequel le rapport du diamètre intérieur (D) du cathéter (45, 145) d'introducteur sur celui du diamètre extérieur (Y) pouvant s'enclencher de la partie de manchon (180) allongée, en prise avec un joint est supérieur à 1,2 à 1 et en particulier supérieur à 1,5 à 1.

6. Ensemble dispositif de mise en place selon l'une quelconque des revendications précédentes, comprenant un dispositif ancrable (70) monté coulissant sur la partie de manchon en prise avec un joint, le dispositif ancrable comportant un joint d'étanchéité (78) qui est apte à entrer en prise étanche avec la partie de manchon (180) allongée, en prise avec un joint.

7. Ensemble dispositif de mise en place selon la revendication 6, dans lequel le dispositif ancrable (70) est mobile d'une première position adjacente distalement à la partie de réception d'un dispositif médical, mais espacée de l'introducteur (40), à une seconde position sur l'extrémité distale de l'introducteur.

8. Ensemble dispositif de mise en place selon la revendication 6 ou 7, dans lequel le dispositif ancrable (70) comprend un mécanisme de verrouillage (74) qui verrouille l'un à l'autre de façon réversible le dispositif ancrable et le dispositif hémostatique (33).

9. Ensemble dispositif de mise en place selon l'une quelconque des revendications précédentes, comprenant un cathéter (11) de fil guide, le cathéter de fil guide s'étendant distalement d'un dilatateur (3) à une poignée à l'extrémité distale du dispositif de mise en place, le cathéter de fil guide pouvant coulisser sur un fil guide (12).

10. Ensemble dispositif de mise en place selon la revendication 9, comprenant un manchon (80) disposé autour du fil guide, le manchon comportant la partie de réception d'un dispositif médical à son extrémité proximale et la partie de manchon allongée, en prise avec un joint étant en position distale par rapport à la partie de réception d'un dispositif médical.
